(19) Europäisches Patentamt
European Patent Office
Office européen des brevets

(11) EP 1 383 515 B1

(12) **EUROPEAN PATENT SPECIFICATION**

(45) Date of publication and mention of the grant of the patent:
**15.12.2004 Bulletin 2004/51**

(21) Application number: **02726402.7**

(22) Date of filing: **11.04.2002**

(51) Int Cl.7: **A61K 31/7076**, A61K 31/52, A61K 31/70

(86) International application number:
**PCT/IL2002/000298**

(87) International publication number:
**WO 2002/083152 (24.10.2002 Gazette 2002/43)**

(54) **ACTIVATION OF NATURAL KILLER CELLS BY ADENOSINE A3 RECEPTOR AGONISTS**

AKTIVIERUNG NATÜRLICHER KILLERZELLEN DURCH ADENOSIN-A3-REZEPTORAGONISTEN

ACTIVATION DE CELLULES K NATURELLES PAR DES AGONISTES DE RECEPTEURS ADENOSINE A3

(84) Designated Contracting States:
**AT BE CH CY DE DK ES FI FR GB GR IE IT LI LU MC NL PT SE TR**

(30) Priority: **12.04.2001 US 832818**

(43) Date of publication of application:
**28.01.2004 Bulletin 2004/05**

(73) Proprietor: **Can-Fite Biopharma Ltd.**
**Petach Tikva 49170 (IL)**

(72) Inventor: **FISHMAN, Pnina**
**46365 Herzliya (IL)**

(74) Representative: **Grünecker, Kinkeldey, Stockmair & Schwanhäusser Anwaltssozietät**
**Maximilianstrasse 58**
**80538 München (DE)**

(56) References cited:
**WO-A-01/19360** **WO-A-99/02143**

- **YAO Y ET AL: "ADENOSINE A3 RECEPTOR AGONISTS PROTECT HL-60 AND U-937 CELLS FROM APOPTOSIS INDUCED BY A3 ANTAGONISTS" BIOCHEMICAL AND BIOPHYSICAL RESEARCH COMMUNICATIONS, ACADEMIC PRESS INC. ORLANDO, FL, US, vol. 232, no. 2, 1997, pages 317-322, XP001035137 ISSN: 0006-291X**
- **FISHMAN P ET AL: "ADENOSINE RECEPTORS: NEW TARGETS FOR CANCER THERAPY AND CHEMOPROTECTION" DRUG DEVELOPMENT RESEARCH, NEW YORK, NY, US, vol. 50, no. 1, May 2000 (2000-05), page 101 XP001003005 ISSN: 0272-4391**
- **BARALDI ET AL: "Synthesis and Biological Activity of a New Series of N6-Arylcarbamoyl, 2-(Ar)alkynyl-N6-arylcarbamoyl, and N6-Carboxamido Derivatives of Adenosine-5'-N-ethyluronamide as A1 and A3 Adenosine Receptor Agonists" JOURNAL OF MEDICINAL CHEMISTRY, AMERICAN CHEMICAL SOCIETY, US, vol. 41, no. 17, 1998, pages 3174-3185, XP002149470 ISSN: 0022-2623**
- **FISHMAN P ET AL: "ADENOSINE ACTS AS AN INHIBITOR OF LYMPHOMA CELL GROWTH: A MAJOR ROLE FOR THE A3 ADENOSINE RECEPTOR" EUROPEAN JOURNAL OF CANCER, PERGAMON PRESS, OXFORD, GB, vol. 36, no. 11, 2000, pages 1452-1458, XP001035229 ISSN: 0959-8049**
- **JACOBSON K A: "Adenosine A3 receptors: novel ligands and paradoxical effects" TRENDS IN PHARMACOLOGICAL SCIENCES, ELSEVIER TRENDS JOURNAL, CAMBRIDGE, GB, vol. 19, no. 5, 1 May 1998 (1998-05-01), pages 184-191, XP004121096 ISSN: 0165-6147**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 383 515 B1

Printed by Jouve, 75001 PARIS (FR)

## Description

### FIELD OF THE INVENTION

This invention relates to the therapeutic use of adenosine A3 receptor agonists for activating natural killer cells.

### PRIOR ART

The following is a list of prior art which is considered to be pertinent for describing the state of the art in the field of the invention.

- Nilsson J. and Olsson AG. Atherosclerosis **53**:77-82, (1984);
- Ward AC, *et al*. Biochem Biophys Res Commun **224**:10-6 (1994);
- Wilson NJ, *et al*. Biochem Biophys Res Commun **244**:475-80 (1998)].
- Giblett *ER, et al*. Lancet **2**:1067-9 (1972)
- Priebe T, *et al*. Cell Immunol **129**:321-8(1990);
- Miller JS, *et al*. J Immunol **162**:7376-82 (1999);
- Hall TJ, *et al*. Clin Exp Immunol **54**:493-500 (1983);
- Trinchieri G. Annu Rev Immunol **13**:251-76 (1995);
- Link *AA, et al*. J Immunol **164**:436-42 (2000);
- Hasko G, *et al. FASEB* **14**:2065-74 (2000);
- Hasko G, *et al*. Eur J Pharmacol **358**:261-8 (1998);

### BACKGROUND OF THE INVENTION

Natural killer cells (NK cells) were first identified in mice due to their capacity to rapidly lyse certain tumor cell targets. These cells are a small subset of peripheral blood lymphocytes, constituting 5 to 16 percent of the total lymphocyte population and form a distinct group of lymphocytes with no immunological memory and are independent of the adaptive immune system.

NK cells mediate a variety of functions that are important in human health and disease. For example, it has been found that NK cells are an important first line of defense against malignant cells and cells infected with viruses, bacteria, and protozoa. In addition, these cells participate in immunoregulation, haematopoiesis, reproduction and neuroendocrine interactions. The finding that NK cells effect the production of a number of cytokines, led to the suggestion that NK cells, like T cells, differentiate into discrete functional subsets with differing effects on adaptive immunity.

### SUMMARY OF THE INVENTION

It was found in accordance with the present that adenosine A3 receptor agonists (A3RAg) activate natural killer (NK) cells and that this activation was abolished in the presence of adenosine A3 receptor antagonists (A3RAn).

Adenosine is a ubiquitous nucleoside present in all body cells. It is released from metabolically active or stressed cells and subsequently acts as a regulatory molecule. It binds to cells through specific A1, $A_{2A}$, $A_{2B}$ and A3 G-protein associated cell surface receptors, thus acting as a signal transduction molecule by regulating the levels of adenylyl cyclase and phospholipase C [Linden J. *The FASEB J* **5**:2668-2676 (1991); Stiles G.L. *Clin. Res.* **38**:10-18 (1990)].

In accordance with a first of its aspects, the present invention concerns the use of adenosine A3 receptor agonists (A3RAg) for the preparation of pharmaceutical compositions for achieving a therapeutic effect, the therapeutic effect comprising activation ofNK cells.

The term ***"adenosine A3 receptor agonist"*** for purposes herein refers to any molecule capable of binding to the adenosine A3 receptor, thereby *fully* or *partially* activating said receptor. Examples for such molecules are provided hereinafter. A *full agonist* for purposes herein should be understood as an agonist, which produces the maximal possible response achievable by activation of its receptor in the preparation being studied, while a *partial agonist* should be understood as an agonist which, no matter how high a concentration is applied, is unable to produce maximal activation of the receptors.

The ***"effective amount"*** (or ***"amount effective for"***) for purposes herein is determined by such considerations as may be known in the art. The amount must be effective to achieve activation of NK cells at a detectable and preferably at a therapeutically effective level. A person versed in the art will know how to determine the effective amount depending, *inter alia,* on the type and severity of the disease to be treated and the treatment regime.

The term ***"activation of NK"*** for purposes herein refers to activation *per se* of the cytotoxic or cytostatic action

of NK cells on foreign or abnormal and undesired cells or elevation of the cytotoxic or cytostatic action of pre-active (i. e. already active) NK cells on the undesired (foreign or abnormal) cells, as well as elevation of their other biological functions, such as stimulation of cytokine production

The present invention also concerns a method for activating NK cells ex vivo by providing said cells with an effective amount of an adenosine A3 receptor agonist (A3RAg).

Further, the present invention refers to the use of A3RAg in an effective amount for the manufacture of a medicament suitable for achieving a therapeutic effect, the therapeutic effect comprises activation ofNK cells in said individual.

The term ***"treatment"*** for the purposes used herein refers to amelioration of undesired symptoms associated with a disease even without curing the disease, e.g. reduction of pain; prevention of the manifestation of such symptoms before they occur; slow down of the deterioration of symptoms or the progression of a disease; lessening of the severity or cure of the disease; acceleration of the natural or conventional healing processes; improvement of survival rate or more rapid recovery of a the individual suffering from a disease; prevention of a disease form occurring or a combination of two or more of the above.

The invention also concerns the use of A3RAg for the manufacture of a medicament suitable for the treatment of a disease or disorder comprising the activation of NK cells, wherein said NK cells are ex vivo activated by contacting NK cells with an effective amount of A3RAg. Typically, such a method comprises withdrawing NK cells from the individual (i.e. autologous NK cells), exposing such cells to an effective amount of at least one A3RAg and introducing the activated NK cells to the recipient (*ex vivo* activation). Alternatively, the NK cells may be withdrawn from a donor. Such donated NK cells may be withdrawn after activation with the A3RAg in primed donor or activated *in vitro* after withdrawal and before administering to the recipient individual. This aspect of the invention also is known by the term "adoptive transfer" according to which an immunity is transferred by transferring immunocompetent cells from a primed donor to a nonimmune recipient.

The term ***"a priori activation"*** refers to activation of NK cells either in a cell or tissue culture or in an animal model wherein the cells, tissue or animal, respectively, are treated with an amount of A3RAg effective for activation of NK cells, and then cells or tissue preparations containing therein said activated NK cells are removed from the culture or from the animal for administration to a nonimmune individual in need thereof. The activated NK cells administered to an individual are preferably, although not exclusively, autologous cells.

Yet further, the present invention provides a pharmaceutical composition for achieving a therapeutic effect, the composition comprising an A3RAg in an amount effective to achieve said therapeutic effect, the therapeutic effect comprising activation of NK cells.

## BRIEF DESCRIPTION OF THE DRAWINGS

In order to understand the invention and to see how it may be carried out in practice, a preferred embodiment will now be described, by way of non-limiting example only, with reference to the accompanying Figures, in which:

**Fig. 1** shows the effect of treatment of an adenosine A3 receptor agonist, Cl-IB-MECA, on human peripheral blood NK cells following incubation of the cells with 10 nM C1-IB-MECA, and the reversed effect obtained in the presence of an adenosine A3 receptor antagonists (MRS1220), as determined by the level of lysis (%Lysis) of the target cells (K562 leukemia cells) by the activated NK cells.

**Fig. 2** shows the effect of various dosages (10-1000µg/kg body weight) of C1-IB-MECA administered orally to mice on the activation of murine NK cells, determined by the percent (from total release) of release of $^{51}$Cr from YAC lymphoma cells labeled with $Na_2[^{51}Cr]O_4$.

**Fig. 3** shows the time dependent stimulatory effect of Cl-IB-MECA on NK cells activity after oral administration of C1-IB-MECA (10µg/kg body weight) to mice for four consecutive days, determined by the percent (from total release) of release of $^{51}$Cr.

**Fig. 4** shows the level of IL-12 in serum samples derived from C1-IB-MECA treated mice (10µg/kg body weight).

**Fig. 5A-5F** show the inhibitory effect of C1-IB-MECA on the development of melanoma and colon carcinoma in mice (Figs. 5A, 5B), with an increase in the level of IL-I2 in the corresponding melanoma or colon carcinoma bearing mice (Figs. 5C, 5D, respectively). Also, an increased NK cell activity (determined by percent (from total release) of $^{51}$Cr release) was exhibited in spleens derived from C1-IB-MECA treated melanoma or colon carcinoma bearing mice in comparison to untreated mice (Figs. 5E, 5F, respectively).

**Fig. 6** shows the inhibitory effect of engrafted, C1- IB-MECA-"activated" NK cells on tumor cell growth in B16-F10 melanoma bearing mice, determined by the number of lung metastatic foci developed after engraftment of the "activated" NK cells to melanoma bearing mice.

## DETAILED DESCRIPTION OF THE INVENTION

As will be shown in the following Examples, C1-IB-MECA, an A3RAg, was found to activate NK cells, to a biologically significant level, both *in vitro* and *in vivo*. Evidently, this finding has a therapeutic value as NK cells participate in a number of biological processes, including defense against malignant and infectious diseases, immunoregulation, haematopoiesis, reproduction and neuroendocrine interactions.

In accordance with a one of its aspects, the present invention provides a method for activating natural killer (NK) cells ex vivo.

The characteristic and methods of preparation of some Adenosine A3 receptor agonists are described in detail in, *inter alia,* US 5,688,774; US 5,773,423, US 5,573,772, US 5,443,836, US 6,048,865, WO 95/02604, WO 99/20284 and WO 99/06053, WO 97/27173, as well as in other publications which are readily available to those versed in the art.

According to one embodiment of the invention, the A3RAg is a compound of the general formula (I):

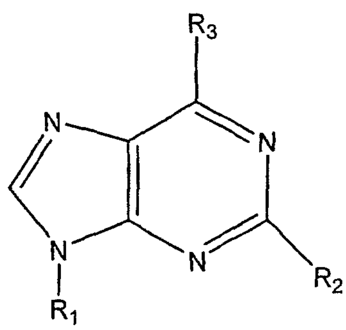

(I)

wherein,

- $R_1$ represents an alkyl, hydroxyalkyl, carboxyalkyl or cyanoalkyl or a group of the following general formula (II):

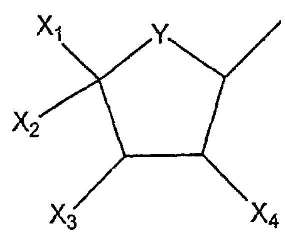

(II)

in which:

- **Y** represents an oxygen, sulfur of carbon atom;
- $X_1$ represents H, alkyl, $R^aR^bNC(=O)$- or $HOR^c$-, wherein
  - $R^a$ and $R^b$ may be the same or different and are selected from hydrogen, alkyl, amino, haloalkyl, aminoalkyl, BOC-aminoalkyl, and cycloalkyl or are joined together to form a heterocyclic ring containing two to five carbon atoms; and
  - $R^c$ is selected from alkyl, amino, haloalkyl, aminoalkyl, BOC-aminoalkyl, and cycloalkyl;
- $X_2$ is H, hydroxyl, alkylamino, alkylamido or hydroxyalkyl;
- $X_3$ and $X_4$ represent independently hydrogen, hydroxyl, amino, amido, azido, halo, alkyl, alkoxy, carboxy, nitrilo, nitro, trifluoro, aryl, alkaryl, thio, thioester, thioether, -OCOPh, -OC(=S)OPh or both $X_3$ and $X_4$ are oxygens connected to >C=S to form a 5-membered ring, or $X_2$ and $X_3$ form the ring of formula (III):

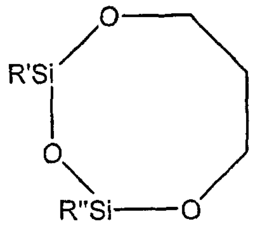

(III)

where **R'** and **R"** represent independently an alkyl group;

- **$R_2$** is selected from hydrogen, halo, alkylether, amino, hydrazido, alkylamino, alkoxy, thioalkoxy, pyridylthio, alkenyl; alkynyl, thio, and alkylthio; and
- **$R_3$** is a group of the formula $-NR_4R_5$ wherein
- **$R_4$** is a hydrogen atom or a group selected from alkyl, substituted alkyl or aryl-NH-C(Z)-, with **Z** being O, S, or $NR^a$ with **$R^a$** having the above meanings; wherein when $R_4$ is hydrogen than
- **$R_5$** is selected from of R- and S-1-phenylethyl, benzyl, phenylethyl groups unsubstituted or substituted in one or more positions with a substituent selected from alkyl, amino, halo, haloalkyl, nitro, hydroxyl, acetoamido, alkoxy, and sulfonic acid or a salt thereof; benzodioxanemethyl, fururyl, L-propylalanyl- aminobenzyl, β-alanylamino- benzyl, T-BOC-β-alanylaminobenzyl, phenylamino, carbamoyl, phenoxy or cycloalkyl; or $R_5$ is a group of the following formula:

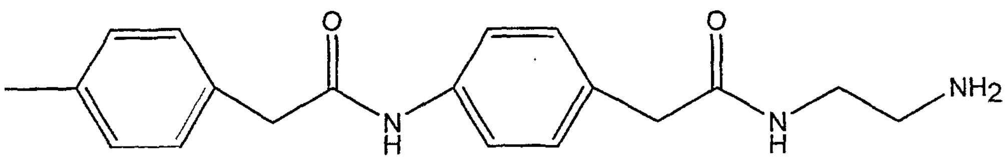

or when **$R_4$** is an alkyl or aryl-NH-C(Z)-, then, **$R_5$** is selected from the group consisting of heteroaryl-$NR^a$-C(Z)-, heteroaryl-C(Z)-, alkaryl-$NR^a$-C(Z)-, alkaryl-C(Z)-, aryl-NR-C(Z)- and aryl-C(Z)-; **Z** representing an oxygen, sulfur or amine; or a physiologically acceptable salt of the above compound.

More preferably, the A3RAg is a nucleoside derivative of the general formula (IV):

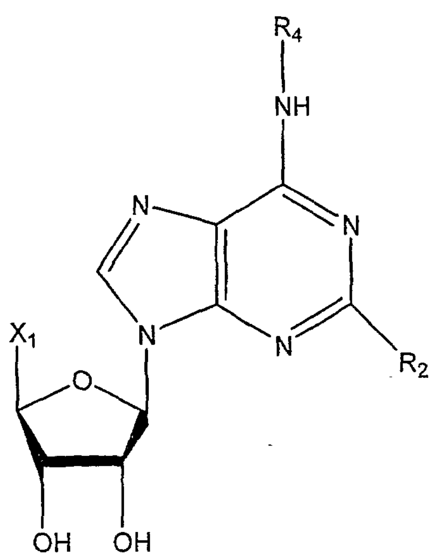

(IV)

and physiologically acceptable salts of said compound, wherein **$X_1$**, **$R_2$** and **$R_4$** are as defmed above.

The non-cyclic aliphatic groups (e.g. alkyl, alkenyl, alkynyl, alkoxy, aralkyl, alkaryl, alkylamine, etc) forming part of the substituent of the compounds of the present invention are either branched or linear aliphatic chains, preferably containing from one or two to twelve carbon atoms.

When referring to ***"physiologically acceptable salts"*** of the compounds employed by the present invention

it is meant any non-toxic alkali metal, alkaline earth metal, and ammonium salts commonly used in the pharmaceutical industry, including the sodium, potassium, lithium, calcium, magnesium, barium ammonium and protamine zinc salts, which are prepared by methods known in the art. The term also includes non-toxic ***acid addition salts,*** which are generally prepared by reacting the compounds of this invention with a suitable organic or inorganic acid. The acid addition salts are those which retain the biological effectiveness and qualitative properties of the free bases and which are not toxic or otherwise undesirable. Examples include, *inter alia*, acids derived from mineral acids, hydrochloric, hydrobromic, sulfuric, nitric, phosphoric, metaphosphoric and the like. Organic acids include, inter alia, tartaric, acetic, propionic, citric, malic, malonic, lactic, fumaric, benzoic, cinnamic, mandelic, glycolic, gluconic, pyruvic, succinic salicylic and arylsulphonic, e.g. p-toluenesulphonic, acids.

Specific examples of A3RAg which may be employed according to general formula (IV) of the present invention include, without being limited thereto, $N^6$-2-(4-aminophenyl)ethyladenosine (APNEA), $N^6$-(4-amino-3-iodobenzyl) adenosine-5'-(N-methyluronamide) (AB-MECA) and $N^6$-(2-iodobenzyl)-adenosine-5'-N-methlyuronamide (IB-MECA) and preferably 2-chloro-$N^6$-(2-iodobenzyl)-adenosine-5'-N-methlyuronamide (C1-IB-MECA).

Further, provided by the present invention is the use of A3RAg for the manufacture of a medicament suitable for the treatment of a disease or disorder, comprising the activation of NK cells, wherein said NK cells are ex vivo activated by contacting NK cells with an activating amount of an A3RAg. In accordance with one embodiment, the NK cells are autologous cells withdrawn from the same individual and then activated *ex vivo* by contacting them with an amount of an A3RAg effective to achieve said activation, and then reintroduced to the individual, by a suitable parenteral administration. Alternatively, the NK cells may be obtained from a donor either after activation *in vivo* by administering the A3RAg to the donor a sufficient time prior to withdrawal of the cells, or activating the cells in a culture as above, or both. Methods for withdrawal of relatively purified NK cells populations from an individual and their treatment in culture are known in the art and need not be further elaborated herein.

The A3RAg may be formulated in different ways. It may be formulated as such or converted into a pharmaceutically acceptable salt. It can be administered alone or in combination with pharmaceutically acceptable carriers, diluents, additives and adjuvants (generally referred to herein as *pharmaceutically acceptable excipients*).

When providing A3RAg to an individual for *in vivo* treatment or to an animal model for *adoptive transfer* treatment, it is preferably formulated for oral delivery. However, other methods of administration are also suitable such as parenteral administration including intravenous, subcutaneous, intramuscular intramedullary injection, intraarterial, intraperitoneally and intranasal administration as well as intrathecal and by infusion techniques. For oral administration, A3RAg with good oral bioavailability is preferably chosen. Screening for an A3RAg with good oral bioavailability and good effectivity in achieving the desired therapeutic effect, is a routine task within easy reach of the artisan.

When administering A3RAg orally, it is preferably formulated for administration as a tablet, a suspension, a solution, an emulsion, a capsule, a powder, a syrup and the like.

When administering A3RAg parenterally, it will generally be formulated in a unit dosage injectable form (solution, suspension, emulsion) and will include sterile aqueous solutions or dispersions and sterile powders for reconstitution into sterile injectable solutions or dispersions.

It is noted that humans are treated generally longer than experimental animals as exemplified herein, which treatment has a length proportional to the length of the disease process. The doses may be single doses or multiple doses over a period of time, e.g. several days and may depend of physical characteristics such as the high, weight, and gender of the individual to be treated. Generally, the administrated doses are preferably unit dosage form. The treatment generally has a length which may be contingent on the length and stage of the disease process and active agent effectiveness and the patient species being treated.

Thus, the present invention also provides pharmaceutical compositions for achieving a therapeutic effect, the composition comprising a pharmaceutically acceptable excipients and an amount of an active ingredient effective to achieve said therapeutic effect, the therapeutic effect comprising activation of NK cells and the active ingredient being an A3RAg. As may be appreciated by those versed in the art, the composition of the invention may contain a single A3RAg or a combination of two or more A3RAg.

By the term ***"pharmaceutically acceptable excipients"*** it is meant any inert, non-toxic materials, which do not react with A3RAg and which are typically added to formulations as diluents or carriers or to give form or consistency to the formulation to give it a specific form, e.g. in pill form, as a simple syrup, aromatic powder, and other various forms. The excipients may also be substances for providing the formulation with stability (e.g. preservatives) or for providing the formulation with an edible flavor etc.

The excipients may be any of those conventionally used and is limited only by chemico-physical considerations, such as solubility and lack of reactivity with A3RAg, and by the route of administration. The choice of excipient will be determined in part by the specific A3RAg employed, as well as by the particular method used to administer the composition. Accordingly, the excipient may include additives, colorants, diluents, buffering agents, disintegrating agents, moistening agents, preservatives, flavoring agents, and pharmacologically compatible carriers. In addition, the excipients may be an adjuvant, which, by definition are substances affecting the action of the active ingredient in a

predictable way.

Accordingly, pharmaceutical compositions suitable for oral administration may consist of (a) liquid solutions, where an effective amount of A3RAg dissolved in diluents, such as water, saline, natural juice, alcohols, syrups, etc.; (b) capsules (e.g. the ordinary hard- or soft-shelled gelatin type containing, for example, surfactants, lubricants, and inert fillers), tablets, lozenges (wherein A3RAg is in a flavor, such as sucrose and acacia or tragacanth or the A3RAg is in an inert base, such as gelatin and glycerin), and troches, each containing a predetermined amount of A3RAg as solids or granules; (c) powders; (d) suspensions in an appropriate liquid; (e) suitable emulsions; (f) liposome formulation; and others.

At times, A3RAg may be made into aerosol formulations to be administered via inhalation. These aerosol formulations can be placed into pressurized acceptable propellants, such as dichlorodifluoromethane, propane, nitrogen, and the like. They also may be formulated as pharmaceuticals for non-pressured preparations, such as in a nebulizer or an atomizer.

Further at times, the pharmaceutical compositions may be formulated for parenteral administration may include aqueous and non-aqueous, isotonic sterile injection solutions, which can contain anti-oxidants, buffers, bacteriostats, and solutes that render the formulation isotonic with the blood of the intended recipient, and aqueous and non-aqueous sterile suspensions that include suspending agents, solubilizers, thickening agents, stabilizers, and preservatives. Oils such as petroleum, animal, vegetable, or synthetic oils and soaps such as fatty alkali metal, ammonium, and triethanolamine salts, and suitable detergents may also be used for parenteral administration. Further, in order to minimize or eliminate irritation at the site of injection, the compositions may contain one or more nonionic surfactants. Suitable surfactants include polyethylene sorbitan fatty acid esters, such as sorbitan monooleate and the high molecular weight adducts of ethylene oxide with a hydrophobic base, formed by the condensation of propylene oxide with propylene glycol.

The parenteral formulations can be presented in unit-dose or multi-dose sealed containers, such as ampoules and vials, and can be stored in a freeze-dried (lyophilized) condition requiring only the addition of the sterile liquid carrier, for example, water, for injections, immediately prior to use. Extemporaneous injection solutions and suspensions can be prepared from sterile powders, granules, and tablets of the kind previously described.

The invention will now be exemplified in the following. It is to be understood that these examples are intended to be in the nature of illustration.

## SPECIFIC EXAMPLES

The effect of the synthetic A3 adenosine receptor agonist 2-chloro-$N^6$-(2-iodobenzyl)- adenosine- 5'-N-methlyuronamide (C1-IB-MECA), on the *in vitro, ex vivo* and *in vivo* activity of NK cells, was tested.

The A3 adenosine receptor antagonist 9-chloro-2-(2-furanyl)-5-[(phenylacetyl)amino] [1,2,4,] -triazolo[1,5-c] quinazoline (MRS-1220) was used to prove the specific binding of C1-IB-MECA to the A3AR.

All drugs were purchased from RBI Massachusetts, USA. A stock solution was prepared by dissolving 5mg C1-IB-MECA or MRS 1220 in dimethylsulfoxide (DMSO). Further dilutions were performed in RPMI.

Murine splenocytes were derived from spleens of ICR mice (Harlan Laboratories, Jerusalem, Israel) and human mononuclear cells were separated by Ficoll-Hypaque gradient from heparinized blood of healthy normal volunteers.

## EXAMPLE 1

The effect of C1-IB-MECA on the activity of human peripheral blood NK cells was assayed by a standard 4h $^{51}$Cr-release assay using K562 leukemia cells as targets. Human mononuclear cells were cultured at a concentration of $5x10^5$ cells/well in 96 well round bottom plates, and used as the effector (E) cells. The cells were preincubated with 10 nM C1-IB-MECA for 18 hours, then the agonist was washed out from the wells and the cells were re-suspended in RPMI containing 10% FCS. K562 cells were used as the targets (T) and were labeled with 100µci of $Na_2[^{51}Cr]O_4$ at 37°C, for 1 hr.

After extensive washing to remove the excess $^{51}$Cr, target cells ($1x10^4$) were re-suspended in RPMI and mixed with the effector cells at an E:T ratio of 1:50 in a total volume of 200 µl (triplicate assays). After 4 hours of incubation at 37°C in 5% $CO_2$, plates were centrifuged, and the supernatants were counted in a gamma counter (LKB).

NK cytotoxicity was calculated using the following equation (CPM: counts per minute):

$$\% \text{ Lysis} = \frac{\text{CPM sample - CPM spontaneous}}{\text{CPM maximal - CPM spontaneous}} \text{ X } 100$$

*CPM spontaneous* and *CPM maximal* were determined by measuring the CPM of the supernatants of the

target cells in the presence of $Na_2[^{51}Cr]O_4$, in the assay medium or in the presence of 1% triton, respectively. It should be noted that spontaneous release was below 10% of the maximal release throughout this experiment.

An increase in the activity of natural killer cells following preincubation with C1-IB-MECA was observed. Introduction of the A3 adenosine receptor antagonist MRS-1220 abolished the stimulatory effect, exhibiting the specific activation of the A3 adenosine receptor by C1-IB-MECA (Figure 1).

**EXAMPLE 2**

Drugs

C1-IB-MECA was dissolved in DMSO and then dilutions were obtained in PBS for the *in vivo* experiments.

*Ex vivo* NK Activity

To examine the induction *in vivo* of NK cells activity by C1-IB-MECA, male ICR mice (Harlan Laboratories, Jerusalem, Israel) aged two months, weighing an average of 20gr, were orally administered with C1-IB-MECA.

Splenocytes derived from the treated mice were removed for evaluation and the NK cell activity was tested by a standard 4h $[^{51}Cr]$-release assay, using YAC lymphoma cells as targets. In particular, the splenocytes ($1x10^6$) were cultured in 96 round bottom well plates and resuspended in RPMI containing 10% FBS. The YAC lymphoma cells were labeled with 100µci of $Na_2[^{51}Cr]O_4$ at 37°C for 1h. $2x10^4$ cells were then resuspended and mixed with the effector cells at the E:T ratio of 50:1, in a volume of 200 µl. After 4h of incubation at 37°C in 5% $CO_2$, plates were centrifuged and supernatants were counted in a gamma counter (LKB). NK cytotoxicity was calculated using the equation described above.

Initially, the dose dependence effect of C1-IB-MECA was determined by administering the mice with various dosages of the drug (10-1000µg/Kg body weight) via the oral route. Splenocytes were separated from the mice 48 hours after treatment with the drug and tested for NK activity using the $^{51}Cr$ assay as described above. As shown in Fig. 2, a dose dependent potentiation of NK activity was induced by C1-IB-MECA and the maximal activity was observed at a dose of 10µg/kg body weight.

In another experiment, the time kinetic effect of C1-IB-MECA (10µg/kg body weight) on NK activity was examined. In this experiment, mice were treated for four consecutive days with the drug. Four, 11 and 18 days after last treatment mice were sacrificed and spleens were removed. Splenocytes were separated and tested for NK activity using the $^{51}Cr$ assay as described above. In addition, serum samples were collected for evaluation of IL-12 levels. Each group contained five mice and the study was repeated four times.

The results produced a bell-shaped response, with NK cell activity peaking at day 4 after last C1-IB-MECA treatment, reaching the control level at day 18 (Fig. 3). An increase in the level of the cytokine IL-12 was noted in serum samples derived from C1-IB-MECA treated mice (Fig. 4).

**EXAMLE 3**

Tumor Cells

B16-F10, melanoma cells and HCT-116, human colon carcinoma cells were used in this set of experiments. The cells were maintained in RPMI medium containing 10% Fetal Bovine Serum (FBS). Cells were transferred to a freshly prepared medium twice weekly.

In this *in vivo* study C57B1/6J male mice (Harlan Laboratories, Jerusalem, Israel) aged two months, weighing an average of 25 gr, were used as the artificial melanoma lung metastasis mice model, which will develop metastatic lung foci 15 days after inoculation. The C57B1/6J mice were inoculated intravenously (i.v.) with B-16 melanoma cells ($2.5x10^5$) and treated daily orally with 10µg/kg body weight of C1-IB-MECA (starting one day after tumor inoculation). As control, served mice treated with the vehicle only.

After 15 days, the mice were sacrificed and spleens were removed and serum samples were collected for IL-12 levels evaluation. Each group contained 15 mice and the study was repeated 3 times.

In yet another experiment, HCT-116 ($1.2x10^6$) colon carcinoma cells were subcutaneously injected to the flank Nude/BalbC mice. Mice were treated orally, daily, one day following tumor inoculation with 10µg/Kg body weight of C1-IB-MECA. As control, served mice treated with the vehicle only. Tumor growth was evaluated every 3-4 days, starting 13 days following tumor inoculation, by measuring width (W) and length (L) of the tumor. Each group contained 10 mice and the study was repeated 3 times. The tumor size was calculated according to the following formula:

$$\text{Tumor size} = \frac{(W)^2 xL}{2}$$

In addition, serum samples were collected for IL-12 levels evaluation.

A marked inhibition in the development of lung metastatic foci and colon carcinoma tumor size was observed in C1-IB-MECA treated mice (Figs. 5A, 5B). The level of IL-12 that was measured in serum samples derived from C1-IB-MECA treated mice was 41% higher than the level obtained with the control group, in both tumor bearing models (Figs. 5C and 5D). Also, an increased NK cell activity was exhibited in splenocytes derived from the C1-IB-MECA treated melanoma or colon carcinoma bearing mice in comparison to untreated mice (Figs. 5E and 5F).

**EXAMPLE 4**

Adoptive transfer experiment

For an adoptive transfer experiment, splenocytes derived from B16-F10 melanoma bearing mice treated with 10 µg/kg body weight of C1-IB-MECA were designated as *"activated"* cells and those derived from the control group were designated as *"non-activated"* splenocytes. The capability of *"activated"* splenocytes to act *in vivo* against the melanoma cells was examined. *"Non-activated"* and *"activated"* splenocytes (see definition above) were engrafted to mice that were inoculated 4 days earlier with B16-F10 melanoma cells ($2.5x10^5$). As the control served mice that were inoculated with the B16-F10 melanoma cells however were not engrafted with splenocytes.

The mice were sacrificed on day 15 and their lungs were removed. The number of lung melanoma foci was counted using a Dissecting Microscope. Each group contained 10 mice and the study was repeated 3 times.

As may be viewed from Fig. 6, the mice engrafted with *"activated"* splenocytes showed a marked inhibition in development of lung metastatic foci as compared to the number of foci developed with mice engrafted with "non-activated" splenocytes or with the control group. These results prove that C1-IB-MECA activate NK cells.

IL-12 analysis in serum samples

Serum samples for IL-12 level analysis were obtained and assayed by a commercial murine ELISA kit of R&D systems, Minneapolis MN.

Statistical analysis

The results were statistically evaluated using the Student's t-test. For statistical analysis comparison between the mean value of different experiments was carried out. The criterion for statistical significance was $p<0.05$.

**DISCUSSION**

The results presented herein show that the A3AR agonist, C1-IB-MECA, induces IL-12 production followed by elevated NK cells activity in vivo. Splenocytes obtained from mice treated orally with C1-IB-MECA exerted a dose and time dependent increased NK activity when incubated ex vivo with target cells. Treatment of melanoma or colon carcinoma bearing mice with C1-IB-MECA induced production of IL-12, increased NK cell activity and inhibited tumor development. Moreover, splenocyes derived from C1-IB-MECA treated melanoma bearing mice that were engrafted to mice inoculated with B16-F10 melanoma cells, were able to prevent the development of metastatic lung foci.

Adenosine exerts various effects on the immune system including anti-inflammatory activity, modulation of cytokine production, inhibition of platelet aggregation, induction of erythropoietin production, thus inflection of the lymphocyte function [Nilsson J. and Olsson AG. Atherosclerosis 53:77-82, (1984); Ward AC, *et al*. Biochem Biophys Res Commun **224**:10-6 (1994); and Wilson NJ, Jaworowski A, Ward AC, Hamilton JA. cAMP enhances CSF-1-induced ERK Biochem Biophys Res Commun 244:475-80 (1998)]. Adenosine was clinically linked to the immune system by the observation made in patients with SCID, which lack the enzyme adenosine deaminase, thus are unable to catabolize adenosine [Giblett ER, et al. Lancet 2:1067-9 (1972)]. Purine nucleotides have been reported to have multiple effects on lymphoid cells [Priebe T, et al. Cell Immunol 129:321-8(1990)]. Incubating human NK cells with exstracellular purine nucleotides resulted in a dose-dependent inhibition in the proliferation with no effect on the capacity to lyse tumor target cells [Miller JS, et al. J Immunol 162:7376-82 (1999)]. Dibutyryl cAMP or forskulin (an agent that elevated cAMP level in the cells by activating adenylyl cyclase) were able to exert an inhibitory effect on the cytolytic activity of NK cells toward certain tumor target cells [Hall TJ, *et al.* Clin Exp Immunol **54**:493-500 (1983)]. Results obtained with adenosine receptor agonists suggest a role for the A1 and A2 receptors in regulating murine NK cell activity. Priebe et al. (*supra.*)

reported that in mouse spleen lymphocytes, NK cells activity was inhibited by adenosine receptor A2 agonists, whereas potent A1 receptor agonist are more effective stimulators.

The results presented herein show that administration of a specific A3AR agonist resulted in activation of NK cells in vivo.

IL-12 is a cytokine known to be a central inducer of Th1 responses and cell mediated immunity by induction of NK cell activity and production of tumor necrosis factor [Trinchieri G. Annu Rev Immunol 13:251-76 (1995)]. Previous reports have demonstrated that adenosine analogues inhibited the production of IL-12 by activated human monocytes, via stimulation of A2A receptors and subsequent increase of cAMP [Link AA, et al. J Immunol 164:436-42 (2000)]. Hasko et al. [Hasko G, et al. FASEB 14:2065-74 (2000)] showed that adenosine receptor agonists inhibited the production of IL-12 in peritoneal MQ with the order of potency GCS-21680 (A2AR agonist)>IB-MECA(A3AR agonist) >CCPA (A1AR agonist). These results suggest that A2 adenosine receptors have a dominate role in inhibiting IL-12 production by adenosine agonists. Moreover it has been demonstrated that the effect of IB-MECA was biphasic, i.e. the inhibition of IL-12 production reached its maximum at 10μM, while at 100μM IB-MECA increased the production of IL-12 [Hasko G. et al. supra.]. On the other hand, the same group of Hasko & Szabo demonstrated that IP administration of of IB-MECA (500μg/kg) to mice as a pretreatment to lethal dose of an endotoxin, Lypopolysacchride, improved the survival of the mice. The LPS-treatment caused the appearance of high levels of IL-12 in the plasma, while the IB-MECA pre-treatment suppressed the levels of IL-12 in the plasma. It should be noted that this effect of IB-MECA was only evident when animals were pretreated with the drug and was observed till 8h after treatment [Hasko G, *et al*. Eur J Pharmacol **358**:261-8 (1998)]. In this study, administration of low doses of C1-IB-MECA induced IL-12 production, which was followed by the induction of NK activity. Thus, it may be concluded that A3AR agonist induces IL-12 production, thereby stimulation NK activity which plays a role in C1-IB-MECA anti-tumor effect.

The results presented herein show that oral administration of low doses of A3AR agonist (C1-IB-MECA) induced increased levels of serum IL-12, which was followed by elevated NK cell activity and tumor growth inhibition. These results demonstrate that chronic activation of the receptor could result in an opposite effect then those observed in acute activation of the receptor.

## Claims

**1.** Use of an A3RAg (A3 receptor agonist) for the preparation of a pharmaceutical composition for the treatment of an infectious disease.

**2.** Use according to claim 1 wherein the infectious disease is caused by an agent selected from: a virus, a bacteria and a protozoa.

**3.** The use of Claim 1, wherein said A3RAg is a compound of the general formula (I):

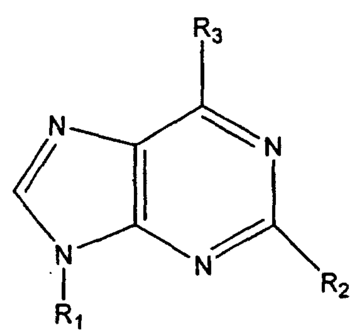

(I)

wherein,

- **$R_1$** represents an alkyl, hydroxyalkyl, carboxyalkyl or cyanoalkyl or a group of the following general formula (II):

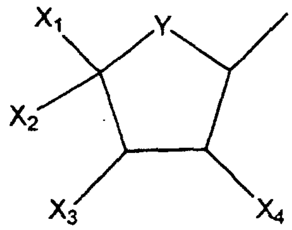

(II)

in which:

- **Y** represents an oxygen, sulfur or $CH_2$;
- $\mathbf{X_1}$ represents H, alkyl, $R^aR^bNC(=O)$- or $HOR^c$-, wherein
  - $\mathbf{R^a}$ and $\mathbf{R^b}$ may be the same or different and are selected from hydrogen, alkyl, amino, haloalkyl, aminoalkyl, BOC-aminoalkyl, and cycloalkyl or are joined together to form a heterocyclic ring containing two to five carbon atoms; and
  - $\mathbf{R^c}$ is selected from alkyl, amino, haloalkyl, aminoalkyl, BOC-aminoalkyl, and cycloalkyl;
- $\mathbf{X_2}$ is H, hydroxyl, alkylamino, alkylamido or hydroxyalkyl;
- $\mathbf{X_3}$ and $\mathbf{X_4}$ represent independently hydrogen, hydroxyl, amino, amido, azido, halo, alkyl, alkoxy, carboxy, nitrilo, nitro, trifluoro, aryl, alkaryl, thio, thioester, thioether, -OCOPh, -OC(=S)OPh or both $\mathbf{X_3}$ and $\mathbf{X_4}$ are oxygens connected to >C=S to form a 5-membered ring, or $\mathbf{X_2}$ and $\mathbf{X_3}$ form the ring of formula (III):

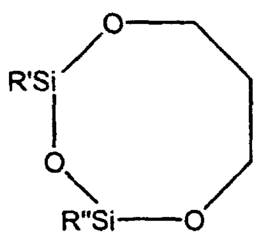

(III)

where **R'** and **R"** represent independently an alkyl group;

- $\mathbf{R_2}$ is selected from hydrogen, halo, alkylether, amino, hydrazido, alkylamino, alkoxy, thioalkoxy, pyridylthio, alkenyl; alkynyl, thio, and alkylthio; and
- $\mathbf{R_3}$ is a group of the formula $-NR_4R_5$ wherein
  - $\mathbf{R_4}$ is a hydrogen atom or a group selected from alkyl, substituted alkyl or aryl-NH-C(Z)-, with **Z** being O, S, or $NR^a$ with $\mathbf{R^a}$ having the above meanings; wherein when $R_4$ is hydrogen than
  - $\mathbf{R_5}$ is selected from R- and S-1-phenylethyl, benzyl, phenylethyl groups unsubstituted or substituted in one or more positions with a substituent s elected from alkyl, amino, halo, haloalkyl, nitro, hydroxyl, acetoamido, alkoxy, and sulfonic acid or a salt thereof; benzodioxanemethyl, fururyl, L-propylalanyl- aminobenzyl, β-alanylamino- benzyl, T-BOC-β-alanylaminobenzyl, phenylamino, carbamoyl, phenoxy or cycloalkyl; or $R_5$ is a group of the following formula:

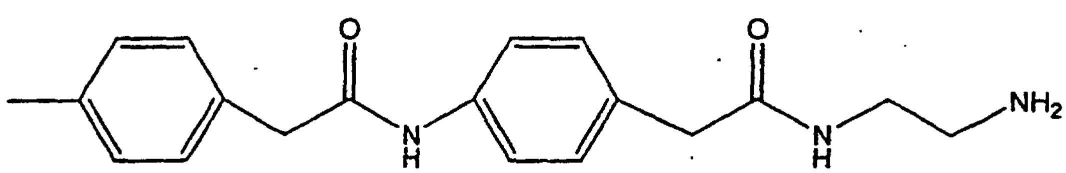

or when $\mathbf{R_4}$ is an alkyl or aryl-NH-C(Z)-, then, $\mathbf{R_5}$ is selected from heteroaryl-$NR^a$-C(Z)-, heteroaryl-C(Z)-, alkaryl-$NR^a$-C(Z)-, alkaryl-C(Z)-, aryl-NR-C(Z)- and aryl-C(Z)-; **Z** representing an oxygen, sulfor or amine; or a pharmaceutically acceptable salt of the above compound.

**4.** The use of Claim 3, wherein said A3RAg is a nucleoside derivative of the general formula (IV):

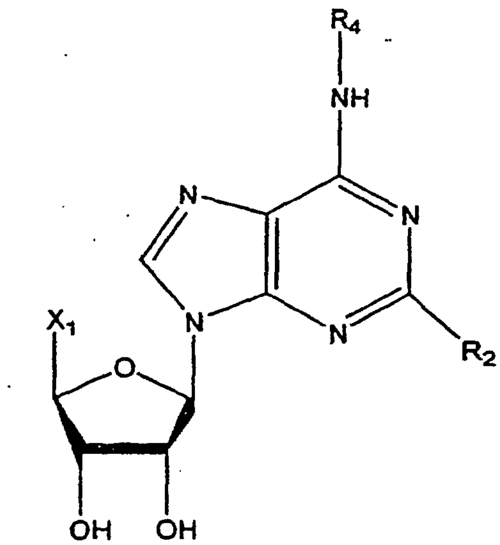

(IV)

wherein $X_1$, $R_2$ and $R_4$ are as defined in Claim 3.

**5.** The use of Claim 1, wherein said A3RAg is selected from $N^6$-2-(4-aminophenyl)ethyladenosine (APNEA), $N^6$-(4-amino-3- iodobenzyl) adenosine-5'-(N-methyl-uronamide) (AB- MECA) , $N^6$-(3-iodobenzyl)-adenosine-5'-N-methyl-uronamide (IB-MECA), and 2-chloro-$N^6$-(3-iodobenzyl)-adenosine-5'-N-methyl-uronamide (C1-IB- MECA).

**6.** The use of Claim 5, wherein said A3RAg is C1-IB-MECA.

**7.** The use of any one of Claims 1-6, for the preparation of a pharmaceutical composition suitable for oral administration.

**8.** A method for activating natural killer (NK) cells comprising contacting said cells *ex vivo* with an amount of an agent effective in activating said NK cells, wherein said active agent is an adenosine A3 receptor agonist (A3RAg).

**9.** The method of Claim 8, wherein said A3RAg is a compound of general formula (I):

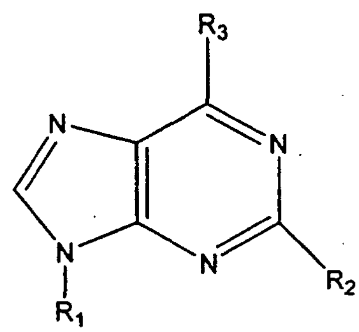

(I)

wherein,

- $R_1$ represents an alkyl, hydroxyalkyl, carboxyalkyl or cyanoalkyl or a group of the following general formula (II):

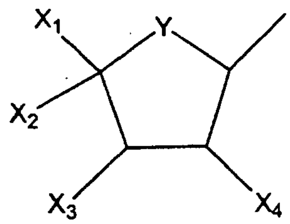

(II)

in which:

- **Y** represents an oxygen, sulfur or $CH_2$;
- $\mathbf{X_1}$ represents H, alkyl, $R^aR^bNC(=O)$- or $HOR^c$-, wherein
  - $\mathbf{R^a}$ and $\mathbf{R^b}$ may be the same or different and are selected from hydrogen, alkyl, amino, haloalkyl, aminoalkyl, BOC-aminoalkyl, and cycloalkyl or are joined together to form a heterocyclic ring containing two to five carbon atoms; and
  - $\mathbf{R^c}$ is selected from alkyl, amino, haloalkyl, aminoalkyl, BOC-aminoalkyl, and cycloalkyl;
- $\mathbf{X_2}$ is H, hydroxyl, alkylamino, alkylamido or hydroxyalkyl;
- $\mathbf{X_3}$ and $\mathbf{X_4}$ represent independently hydrogen, hydroxyl, amino, amido, azido, halo, alkyl, alkoxy, carboxy, nitrilo, nitro, trifluoro, aryl, alkaryl, thio, thioester, thioether, -OCOPh, -OC(=S)OPh or both $\mathbf{X_3}$ and $\mathbf{X_4}$ are oxygens connected to >C=S to form a 5-membered ring, or $\mathbf{X_2}$ and $\mathbf{X_3}$ form the ring of formula (III):

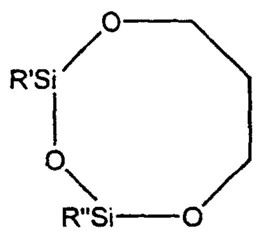

(III)

where **R'** and **R"** represent independently an alkyl group;

- $\mathbf{R_2}$ is selected from hydrogen, halo, alkylether, amino, hydrazido, alkylamino, alkoxy, thioalkoxy, pyridylthio, alkenyl; alkynyl, thio, and alkylthio; and
- $\mathbf{R_3}$ is a group of the formula $-NR_4R_5$ wherein
- $\mathbf{R_4}$ is a hydrogen atom or a group selected from alkyl, substituted alkyl or aryl-NH-C(Z)-, with **Z** being O, S, or $NR^a$ with $\mathbf{R^a}$ having the above meanings; wherein when $R_4$ is hydrogen than
- $\mathbf{R_5}$ is selected from R- and S-1-phenylethyl, benzyl, phenylethyl groups unsubstituted or substituted in one or more positions with a substituent selected from alkyl, amino, halo, haloalkyl, nitro, hydroxyl, acetoamido, alkoxy, and sulfonic acid or a salt thereof; benzodioxanemethyl, fururyl, L-propylalanyl- aminobenzyl, β-alanylamino- benzyl, T-BOC-β-alanylaminobenzyl, phenylamino, carbamoyl, phenoxy or cycloalkyl; or $R_5$ is a group of the following formula:

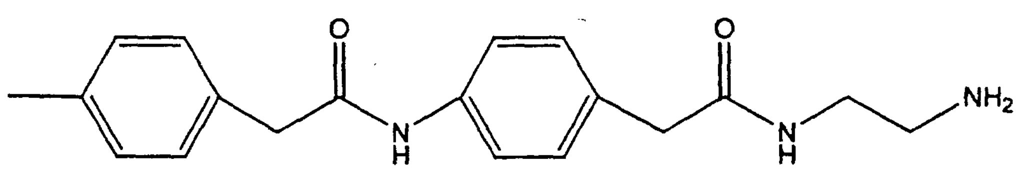

or when $\mathbf{R_4}$ is an alkyl or aryl-NH-C(Z)-, then, $\mathbf{R_5}$ is selected from the group consisting of heteroaryl-$NR^a$-C(Z)-, heteroaryl-C(Z)-, alkaryl-$NR^a$-C(Z)-, alkaryl-C(Z)-, aryl-NR-C(Z)- and aryl-C(Z)-; **Z** representing an oxygen, sulfur or amine;
or a pharmaceutically acceptable salt of the above compound.

**10.** The method of Claim 9, wherein said A3RAg is a compound of general formula (IV):

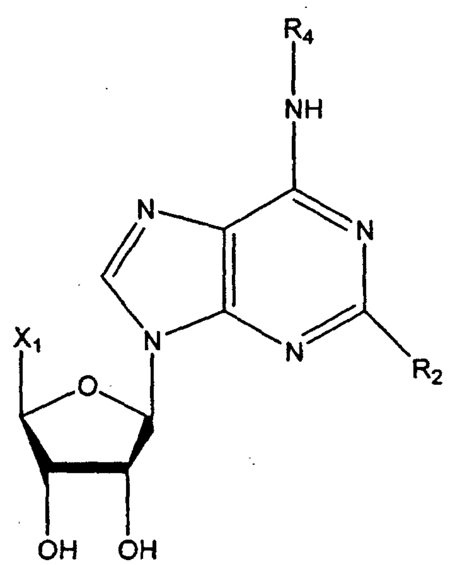

(IV)

wherein $X_1$, $R_2$ and $R_4$ are as defined in Claim 9.

**11.** The method of Claim 10, wherein A3RAg is selected from $N^6$-2-(4-aminophenyl)ethyladenosine (APNEA), $N^6$-(4-amino-3- iodobenzyl) adenosine-5'-(N-methyl-uronamide) (AB-MECA), $N^6$- (3-iodobenzyl)-adenosine-5'-N-methyl-uronamide (IB-MECA), and 2-chloro-$N^6$- (3-iodobenzyl)-adenosine-5'-N- methyl methyl-uronamide (C1-IB-MECA).

**12.** The method of Claim 11, wherein said A3RAg is C1-IB-MECA.

**Patentansprüche**

**1.** Verwendung von A3RAg (A3 receptor agonist) zur Herstellung einer pharmazeutischen Zusammensetzung zur Behandlung einer infektiösen Erkrankung.

**2.** Verwendung gemäß Anspruch 1, wobei die infektiöse Erkrankung durch ein Agens verursacht wird, ausgewählt aus: einem Virus, einem Bakterium und einem Einzeller.

**3.** Die Verwendung gemäß Anspruch 1, wobei das genannte A3RAg eine Verbindung der allgemeinen Formel (I) ist:

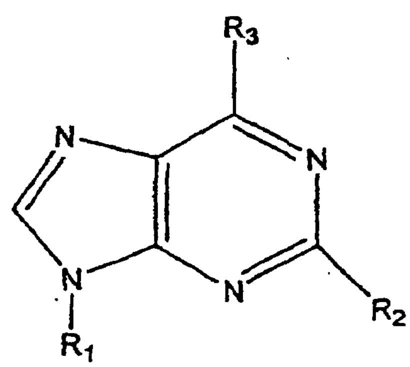

(I)

wobei,

- $R_1$ ein Alkyl, Hydroxyalkyl, Carboxyalkyl oder Cyanoalkyl oder eine Gruppe der folgenden allgemeinen Formel (II) repräsentiert:

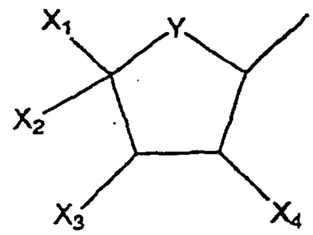

(II)

in welcher:

- Y Sauerstoff, Schwefel oder $CH_2$ repräsentiert;
- $X_1$ ein H, Alkyl, $R^aR^bNC(=O)$- oder $HOR^c$- repräsentiert, wobei
- $R^a$ und $R^b$ gleich oder unterschiedlich sein können und ausgewählt werden aus Wasserstoff, Alkyl, Amino, Haloalkyl, Aminoalkyl, BOC-Aminoalkyl, und Cycloalkyl, oder so miteinander verbunden sind, dass sie einen heterozyklischen Ring bilden, der zwei bis fünf Kohlenstoffatome enthält; und
- $R^c$ ausgewählt wird aus Alkyl, Amino, Haloalkyl, Aminoalkyl, BOC-Aminoalkyl, und Cycloalkyl;
- $X_2$ ein H, Hydroxyl, Alkylamino, Alkylamido oder Hydroxyalkyl ist;
- $X_3$ und $X_4$ unabhängig voneinander Wasserstoff, Hydroxyl, Amino, Amido, A-zido, Halo, Alkyl, Alkoxy, Carboxy, Nitrilo, Nitro, Trifluoro, Aryl, Alkaryl, Thio, Thioester, Thioether, -OCOPh, -OC(=S)OPh darstellen oder beiderseits $X_3$ und $X_4$ Sauerstoff sind, die mit >C=S so verbunden sind, dass sie einen 5-gliedrigen Ring bilden, oder $X_2$ und $X_3$ einen Ring der Formel (III) bilden:

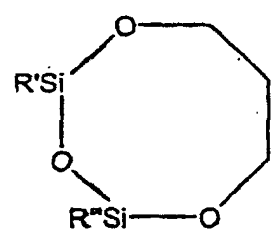

(III)

wobei R' und R" unabhängig voneinander eine Alkylgruppe repräsentieren;

- $R_2$ ausgewählt wird aus Wasserstoff, Halo, Alkylether, Amino, Hydrazido, Alkylamino, Alkoxy, Thioalkoxy, Pyridylthio, Alkenyl; Alkynyl, Thio und Alkylthio; und
- $R_3$ eine Gruppe aus der Formel $-NR_4R_5$ ist, wobei

  - $R_4$ ein Wasserstoffatom oder eine Gruppe ist ausgewählt aus Alkyl, substituiertem Alkyl oder Aryl-NH-C(Z)-, wobei Z, O, S, oder $NR^a$ ist, wobei $R^a$ die oben beschriebenen Bedeutungen besitzt; und im Falle, dass $R_4$ Wasserstoff ist;
  - $R_5$ ausgewählt wird aus R- und S-1-Phenylethyl, Benzyl, Phenylethylgruppen, die unsubstituiert sind oder in einer oder mehreren Positionen mit einem Substituenten substituiert sind, der ausgewählt wird aus Alkyl, Amino, Halo, Haloalkyl, Nitro, Hydroxyl, Acetoamido, Alkoxy und Sulfonsäure oder einem Salz davon; Benzodioxanmethyl, Fururyl, L-Propylalanyl-Aminobenzyl, β-Alanylamino-Benzyl, T-BOC-β-Alanylaminobenzyl, Phenylamino, Carbamoyl, Phenoxy oder Cycloalkyl; oder $R_5$ ist eine Gruppe der folgenden Formel:

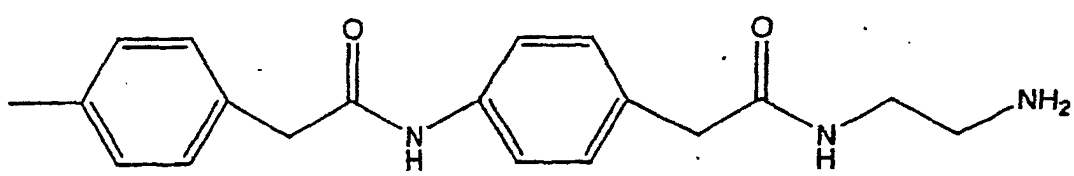

oder, wenn $R_4$ ein Alkyl oder Aryl-NH-C(Z)- ist, wird $R_5$ ausgewählt aus Heteroaryl-$NR^a$-C(Z)-, Heteroaryl-C(Z)-, Alkylaryl-$NR^a$-C(Z)-, Alkaryl-C(Z)-, Aryl-NR-C(Z)- und Aryl-C(Z)-; Z repräsentiert Sauerstoff, Schwefel oder Amin; oder ein pharmazeutisch geeignetes Salz der oben genannten Verbindung.

**4.** Die Verwendung gemäß Anspruch 3, wobei A3RAg ein Nucleosidderivat der allgemeinen Formel (IV) ist:

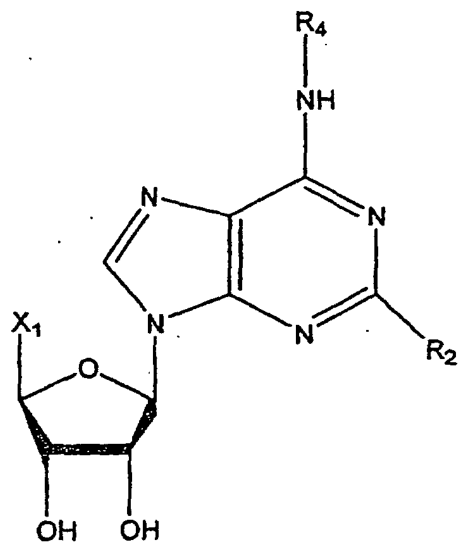

(IV)

wobei $X_1$, $R_2$ und $R_4$ wie in Anspruch 3 definiert sind.

**5.** Die Verwendung gemäß Anspruch 1, wobei das genannte A3RAg ausgewählt wird aus $N^6$-2-(4-Aminophenyl) ethyladenosin (APNEA), $N^6$-(4-Amino-3-lodobenzyl)-Adenosin-5'-(N-Methyl-Uronamid) (AB-MECA), $N^6$-(3-lodobenzyl)-Adenosin-5'N-Methyl-Uronamid (IB-MECA), und 2-Chloro-$N^6$-(3-lodobenzyl)-Adenosin-5'N-Methyl-Uronamid (Cl-IB-MECA).

**6.** Die Verwendung gemäß Anspruch 5, wobei das genannte A3RAg C1-IB-MECA ist.

**7.** Die Verwendung gemäß einem der Ansprüche 1 bis 6 zur Herstellung einer pharmazeutischen Zusammensetzung, die für die orale Verabreichung geeignet ist.

**8.** Ein Verfahren zur Aktivierung natürlicher Killer (NK) Zellen, welches das Kontaktieren der genannten Zellen *ex vivo* mit einer solchen Menge an Agens umfasst, die für die Aktivierung der genannten NK-Zellen wirksam ist, wobei das genannte aktive Agens ein Adenosin A3 Rezeptoragonist (A3RAg) ist.

**9.** Das Verfahren gemäß Anspruch 8, wobei das genannte A3RAg eine Verbindung der allgemeinen Formel (I) ist:

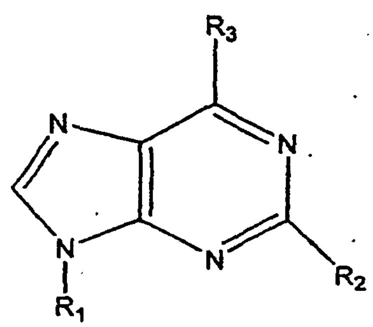

(I)

wobei,

- $R_1$ ein Alkyl, Hydroxyalkyl, Carboxyalkyl oder Cyanoalkyl oder eine Gruppe der folgenden allgemeinen Formel (II) repräsentiert:

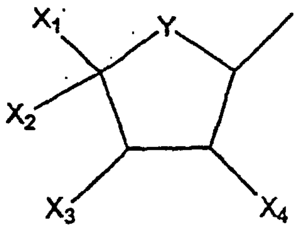

(II)

in welcher:

- Y einen Sauerstoff, Schwefel oder $CH_2$ repräsentiert;
- $X_1$ ein H, Alkyl, $R^aR^bNC(=O)$- oder $HOR^c$- repräsentiert, wobei
- $R^8$ und $R^b$ gleich oder unterschiedlich sein können und ausgewählt werden aus Wasserstoff, Alkyl, Amino, Haloalkyl, Aminoalkyl, BOC-Aminoalkyl und Cycloalkyl, oder miteinander verbunden sein können,um einen heterozyklischen Ring zu bilden, der zwei bis fünf Kohlenstoffatome enthält; und
- $R^c$ ausgewählt wird aus Alkyl, Amino, Haloalkyl, Aminoalkyl, BOC-Aminoalkyl, und Cycloalkyl;
- $X_2$ ein H, Hydroxyl, Alkylamino, Alkylamido oder Hydroxyalkyl ist;
- $X_3$ und $X_4$ unabhängig voneinander Wasserstoff, Hydroxyl, Amino, Amido, Azido, Halo, Alkyl, Alkoxy, Carboxy, Nitrilo, Nitro, Trifluoro, Aryl, Alkaryl, Thio, Thioester, Thioether, -OCOPh, -OC(=S)OPh repräsentieren, oder beiderseits $X_3$ und $X_4$ Sauerstoffe sind, die mit >C=S verknüpft sind, um einen fünfgliedrigen Ring zu bilden, oder $X_2$ und $X_3$ den Ring der Formel (III) bilden:

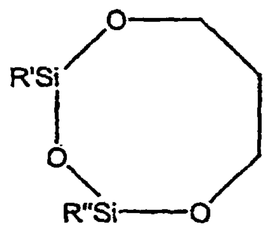

(III)

wobei R' und R" unabhängig voneinander eine Alkylgruppe repräsentieren;

- $R_2$ ausgewählt wird aus Wasserstoff, Halo, Alkylether, Amino, Hydrazido, Alkylamino, Alkoxy, Thioalkoxy, , Pyridylthio, Alkenyl, Alkynyl, Thio und Alkylthio; und
- $R_3$ eine Gruppe der Formel-$NR_4R_5$ ist, wobei
- $R_4$ ein Wasserstoffatom oder eine Gruppe ist ausgewählt aus Alkyl, substituiertem Alkyl oder Aryl-NH-C(Z)-, wobei Z ein O, S oder $NR^a$ ist, wobei $R^a$ die oben genannten Bedeutungen besitzt; wobei im Falle, dass $R_4$ Wasserstoff ist,
- $R_5$ ausgewählt wird aus R- und S-1-Phenylethyl, Benzyl, Phenylethylgruppen, die unsubstituiert sind oder an einer oder mehreren Positionen mit einem Substituenten substituiert sind, der ausgewählt wird aus Alkyl, Amino, Halo, Haloalkyl, Nitro, Hydroxyl, Acetoamido, Alkoxy, und Sulfonsäure, oder einem Salz davon; Benzodioxanmethyl, Fururyl, L-Propylalanyl-Aminobenzyl, β-Alanylamino-Benzyl, T-BOC-β-Alanylaminobenzyl, Phenylamino, Carbamoyl, Phenoxy oder Cycloalkyl; oder $R_5$ ist eine Gruppe der folgenden Formel:

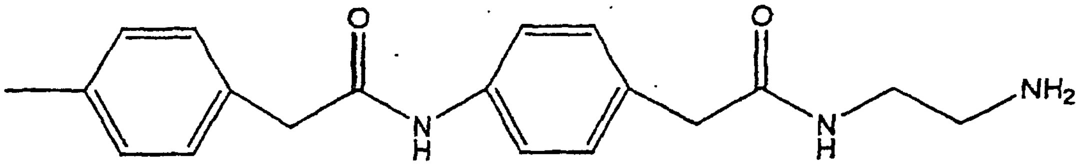

oder wenn $R_4$ ein Alkyl oder Aryl-NH-C(Z)- ist, wird $R_5$ ausgewählt aus der Gruppe bestehend aus Heteroaryl-$NR^a$-C(Z)-, Heteroaryl-C(Z)-, Alkaryl-$NR^a$-C(Z)-, Alkaryl-C(Z)-, Aryl-NR-C(Z)- und Aryl-C(Z)-; Z repräsentiert

Sauerstoff, Schwefel oder Amin;
oder ein pharmazeutisch geeignetes Salz der oben genannten Verbindung.

**10.** Das Verfahren gemäß Anspruch 9, wobei das genannte A3RAg eine Verbindung der allgemeinen Formel (IV) ist:

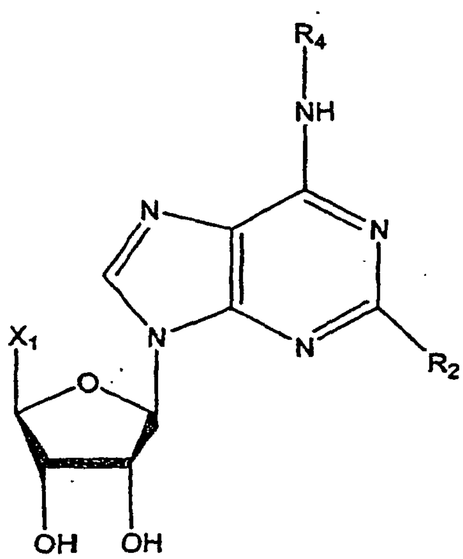

(IV)

wobei $X_1$, $R_2$ und $R_4$ wie im Anspruch definiert sind.

**11.** Das Verfahren gemäß Anspruch 10, wobei A3RAg ausgewählt wird aus $N^6$-2-(4-Aminophenyl)Ethyladenosin (APNEA), $N^6$-(4-Amino-3-Iodobenzyl)-Adenosin-5'-(N-Methyl-Uronamid) (AB-MECA), $N^6$-(3-Iodobenzyl)-Adenosin-5'N-Methyl-Uronamid (IB-MECA), und 2-Chloro-$N^6$-(3-Iodobenzyl)-Adenosin-5'N-Methyl-Uronamid (C1-IB-MECA).

**12.** Das Verfahren gemäß Anspruch 11, wobei das genannte A3RAg C1-IB-MECA ist.

**Revendications**

**1.** Utilisation d'un A3RAg (agoniste du récepteur A3) pour la préparation d'une composition pharmaceutique pour le traitement d'une maladie infectieuse.

**2.** Utilisation selon la revendication 1, dans laquelle la maladie infectieuse est causée par un agent pathogène parmi : un virus, une bactérie et un protozoaire.

**3.** Utilisation selon la revendication 1, dans laquelle ledit A3RAg est un composé de formule générale (I) :

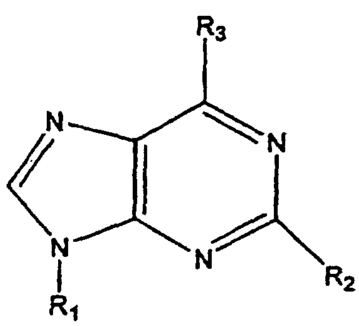

(I)

dans laquelle,

- $R_1$ représente un groupe alkyle, hydroxyalkyle, carboxyalkyle ou cyanoalkyle, ou un groupe de formule générale (II) suivante :

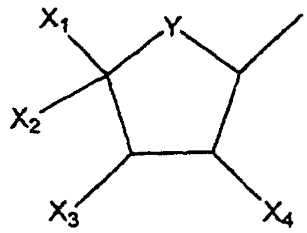

(II)

dans laquelle :

- Y représente un atome d'oxygène, de soufre ou un groupe $CH_2$ ;
- $X_1$ représente un atome d'hydrogène, un groupe alkyle, $R^aR^bNC(=O)$ ou $HOR^c$-, dans lesquels

  - $R^a$ et $R^b$ peuvent être identiques ou différents et sont choisis parmi un atome d'hydrogène, un groupe alkyle, amino, halogénoalkyle, aminoalkyle, BOC-aminoalkyle et cycloalkyle ou sont reliés ensemble pour former un hétérocycle contenant deux à cinq atomes de carbone ; et
  - $R^C$ est choisi parmi un groupe alkyle, amino, halogénoalkyle, aminoalkyle, BOC-aminoalkyle et cycloalkyle ;

- $X_2$ représente un atome d'hydrogène, un groupe hydroxyle, alkylamido ou hydroxyalkyle ;
- $X_3$ et $X_4$ représentent, indépendamment, un atome d'hydrogène, un groupe hydroxyle, amino, amido, azido, halogéno, alkyle, alcoxy, carboxyle, nitrilo, nitro, trifluoro, aryle, alkaryle, thio, thioester, thioéther, -OCOPh, -OC (=S) OPh ou $X_3$ et $X_4$ sont des atomes d'oxygène reliés à >C=S pour former un cycle à cinq éléments, ou $X_2$ et $X_3$ forment le cycle de formule (III) :

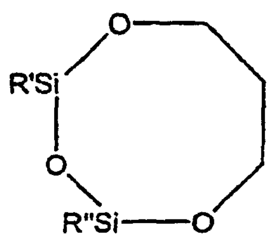

(III)

dans laquelle R' et R" représentent, indépendamment, un groupe alkyle ;

- $R_2$ est choisi parmi un atome d'hydrogène, d'halogène, un groupe éther alkylique, amino, hydrazido, alkylamino, alcoxy, thioalcoxy, pyridylthio, alcényle ; alcynyle, thio et alkylthio ; et
- $R_3$ représente un groupe de formule $-NR_4R_5$ dans laquelle

  - $R_4$ représente un atome d'hydrogène ou un groupe choisi parmi un groupe alkyle, alkyle substitué ou aryl-NH-C(Z)-, où Z représente O, S ou $NR^a$ pour lequel $R^a$ a les significations ci-dessus ; dans laquelle lorsque $R_4$ est un atome d'hydrogène alors
  - $R_5$ est choisi parmi les groupes R- et S-1-phényléthyle, benzyle, phényléthyle non substitués ou substitués dans une ou plusieurs positions par un substituant choisi parmi un groupe alkyle, amino, halogéno, halogénoalkyle, nitro, hydroxyle, acétoamido, alcoxy et acide sulfonique ou un de ses sels ; benzodioxaneméthyle, fururyle, L-propylalanylaminobenzyle, bêta-alanylaminobenzyle, t-BOC-bêta-alanylaminobenzyle, phénylamino, carbamoyle, phénoxy ou cycloalkyle ; ou $R_5$ représente un groupe de formule suivante :

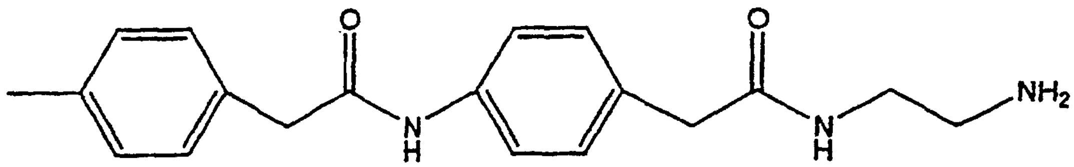

ou lorsque $R_4$ représente un groupe alkyle ou aryl-NH-C(Z)-, alors $R_5$ est choisi parmi un groupe hétéroaryl-$NR^a$-C(Z)-, hétéroaryl-C(Z)-, alkaryl-$NR^a$-C(Z)-, alkaryl-C(Z)-, aryl-MR-C(Z)- ; Z représentant un atome d'oxygène, de souffre ou un groupe amino ;
ou un sel acceptable sur le plan pharmaceutique du composant ci-dessus.

**4.** Utilisation selon la revendication 3, dans laquelle ledit A3RAg est un dérivé nucléoside de formule générale (IV) :

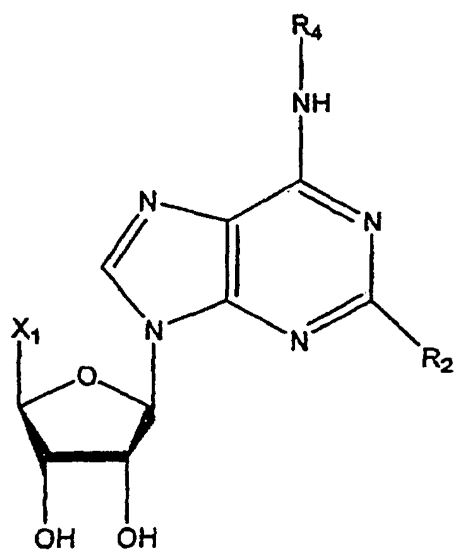

(IV)

dans laquelle $X_1$, $R_2$ et $R_4$ sont tels que définis dans la revendication 3.

**5.** Utilisation selon la revendication 1, dans laquelle ledit A3RAg est choisi parmi la $N^6$-2-(4-aminophényl)éthyladénosine (APNEA), le $N^6$-(4-amino-3-iodobenzyl)adénosine-5'-(N-méthyl-uronamide) (AB-MECA), le $N^6$-(3-iodobenzyl)-adénosine-5'-N-méthyl-uronamide (IB-MECA), et le 2-chloro-$N^6$-(3-iodobenzyl)-adénosine-5'-N-méthyl-uronamide (C1-IB-MECA).

**6.** Utilisation selon la revendication 5, dans laquelle ledit A3RAg est C1-IB-MECA.

**7.** Utilisation selon l'une quelconque des revendications 1 à 6, pour la préparation d'une composition pharmaceutique appropriée à une administration par voie orale.

**8.** Procédé pour l'activation de cellules tueuses naturelles (NK) qui comprend la mise en contact desdites cellules *ex vivo* avec un agent en quantité efficace pour activer lesdites cellules NK, dans lequel ledit agent actif est un agoniste du récepteur adénosine A3 (A3RAg).

**9.** Procédé selon la revendication 8, dans lequel ledit A3RAg est un composé de formule générale (I) :

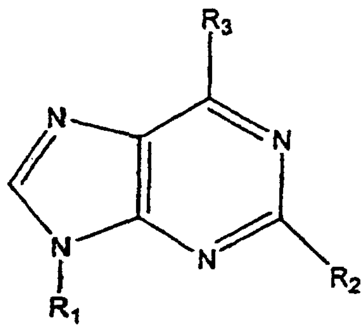

(I)

dans laquelle,

- $R_1$ représente un groupe alkyle, hydroxyalkyle, carboxyalkyle ou cyanoalkyle, ou un groupe de formule générale (II) suivante :

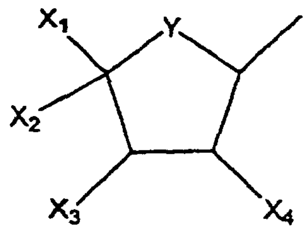

(II)

dans laquelle :

- Y représente un atome d'oxygène, de soufre ou un groupe $CH_2$ ;
- $X_1$ représente un atome d'hydrogène, un groupe alkyle, $R^aR^bNC(=O)$ ou $HOR^c$-, dans lesquels
  - $R^a$ et $R^b$ peuvent être identiques ou différents et sont choisis parmi un atome d'hydrogène, un groupe alkyle, amino, halogénoalkyle, aminoalkyle, BOC-aminoalkyle et cycloalkyle, ou sont reliés ensemble pour former un hétérocycle contenant deux à cinq atomes de carbone ; et
  - $R^C$ est choisi parmi un groupe alkyle, amino, halogénoalkyle, aminoalkyle, BOC-aminoalkyle et cycloalkyle ;
- $X_2$ représente un atome d'hydrogène, un groupe hydroxyle, alkylamido ou hydroxyalkyle ;
- $X_3$ et $X_4$ représentent, indépendamment, un atome d'hydrogène, un groupe hydroxyle, amino, amido, azido, halogéno, alkyle, alcoxy, carboxyle, nitrilo, nitro, trifluoro, aryle, alkaryle, thio, thioester, thioéther, -OCOPh, -OC(=S)OPh, ou $X_3$ et $X_4$ sont tout deux des atomes d'oxygène reliés à >C=S pour former un cycle à cinq éléments, ou $X_2$ et $X_3$ forment le cycle de formule (III) :

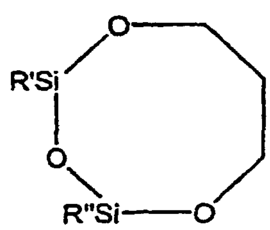

(III)

dans laquelle R' et R" représentent, indépendamment, un groupe alkyle ;

- $R_2$ est choisi parmi un atome d'hydrogène, d'halogène, un groupe éther alkylique, amino, hydrazido, alkylamino, alcoxy, thioalcoxy, pyridylthio, alcényle ; alcynyle, thio et alkylthio ; et
- $R_3$ représente un groupe de formule $-NR_4R_5$ dans laquelle
  - $R_4$ représente un atome d'hydrogène ou un groupe choisi parmi un groupe alkyle, alkyle substitué ou

aryl-NH-C(Z)-, où Z représente O, S ou $NR^a$ avec $R^a$ ayant les significations ci-dessus ; dans laquelle lorsque $R_4$ est un hydrogène, alors

- $R_5$ est choisi parmi les groupes R- et S-1-phényléthyle, benzyle, phényléthyle non substitués ou substitués dans une ou plusieurs positions par un substituant choisi parmi un groupe alkyle, amino, halogéno, halogénoalkyle, nitro, hydroxyle, acétoamido, alcoxy et acide sulfonique ou un de ses sels ; benzodioxaneméthyle, fururyle, L-propylalanylaminobenzyle, bêta-alanylaminobenzyle, T-BOC-bêta-alanylaminobenzyle, phénylamino, carbamoyle, phénoxy ou cycloalkyle ; ou $R_5$ représente un groupe de formule suivante :

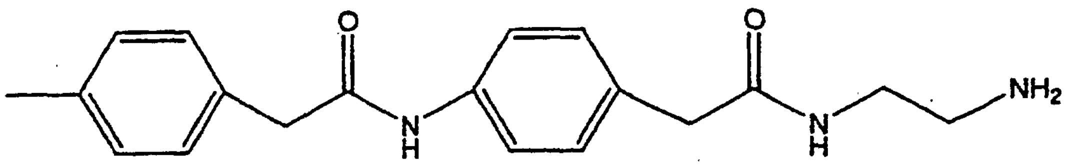

ou lorsque $R_4$ représente un groupe alkyle ou aryl-NH-C(Z)-, alors $R_5$ est choisi dans le groupe composé des groupes hétéroaryl-$NR^a$-C(Z)-, hétéroaryl-C(Z)-, alkaryl-$NR^a$-C(Z)-, alkaryl-C(Z)-, aryl-NR-C(Z)- et aryl-C(Z)- ; Z représentant un atome d'oxygène, de souffre ou un groupe amine ;
ou un sel acceptable sur le plan pharmaceutique du composant ci-dessus.

**10.** Procédé selon la revendication 9, dans lequel ledit A3RAg est un composé de formule générale (IV) :

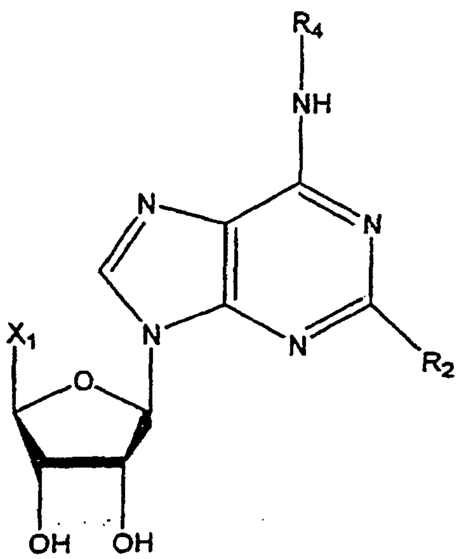

(IV)

dans laquelle $X_1$, $R_2$ et $R_4$ sont tels que définis dans la revendication 3.

**11.** Procédé selon la revendication 10, dans lequel A3RAg est choisi parmi la $N^6$-2-(4-aminophényl)éthyladénosine (APNEA), le $N^6$-(4-amino-3-iodobenzyl)adénosine-5'-(N-méthyl-uronamide) (AB-MECA), le $N^6$-(3-iodobenzyl)-adénosine-5'-N-méthyl-uronamide (IB-MECA), et le 2-chloro-$N^6$-(3-iodobenzyl)-adénosine-5'-N-méthyl-uronamide (C1-IB-MECA) .

**12.** Procédé selon la revendication 11, dans lequel ledit A3RAg est C1-IB-MECA.

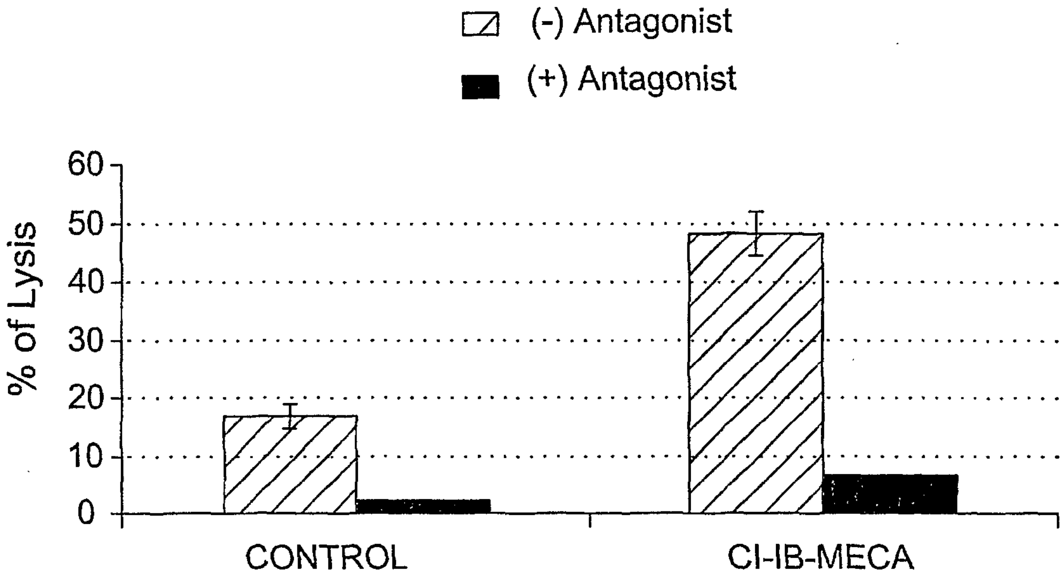
(-) Antagonist
(+) Antagonist
% of Lysis
60
50
40
30
20
10
0
CONTROL
Cl-IB-MECA

FIG. 1

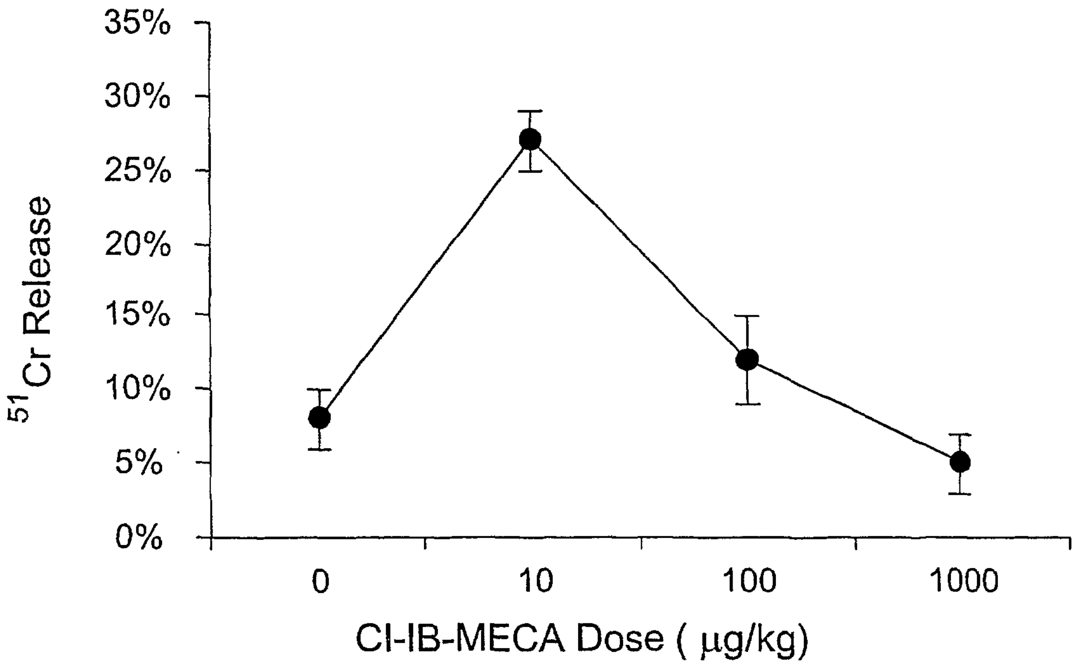
35%
30%
25%
20%
15%
10%
5%
0%
51Cr Release
0
10
100
1000
Cl-IB-MECA Dose ( µg/kg)

FIG. 2

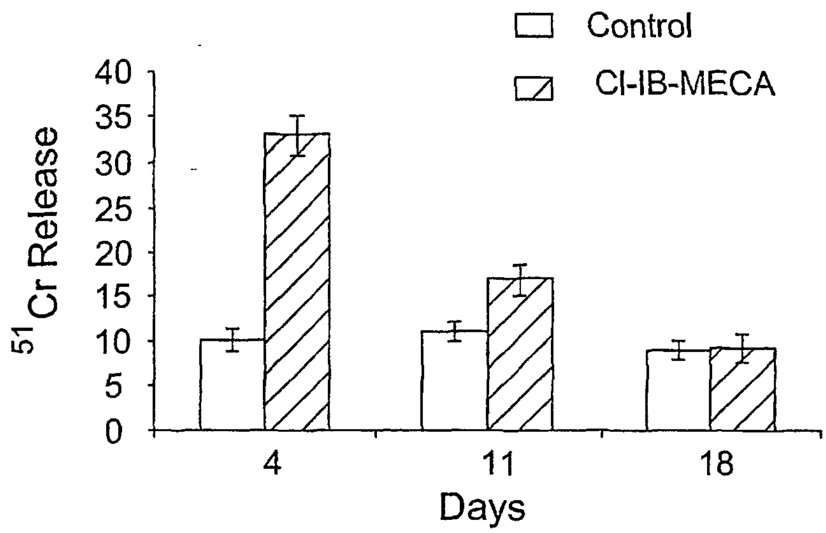
Control
Cl-IB-MECA
40
35
30
25
20
15
10
5
0
$^{51}$Cr Release
4
11
18
Days

FIG. 3

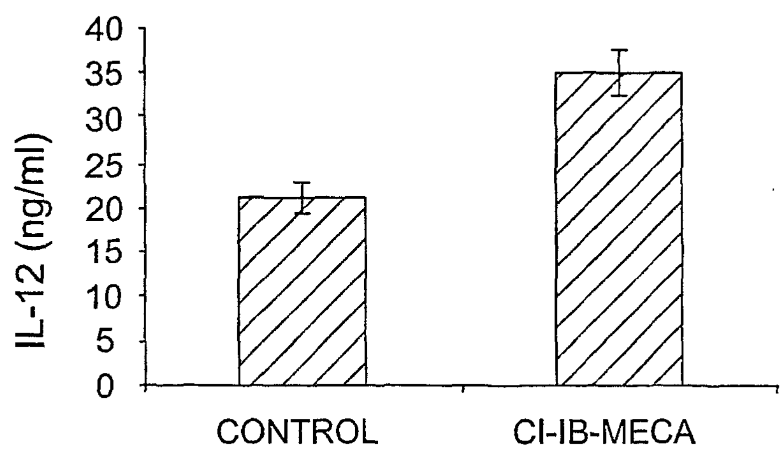
40
35
30
25
20
15
10
5
0
IL-12 (ng/ml)
CONTROL
Cl-IB-MECA

FIG. 4

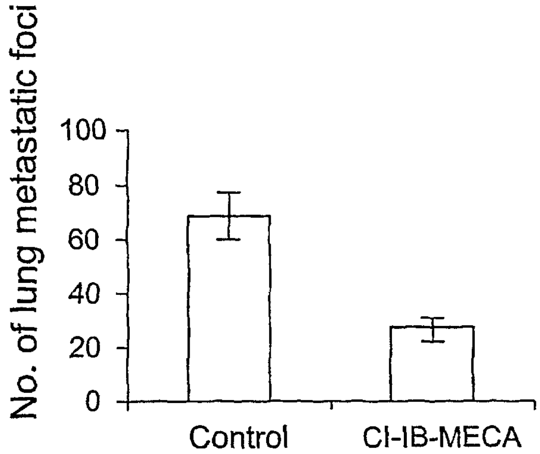


FIG. 5A

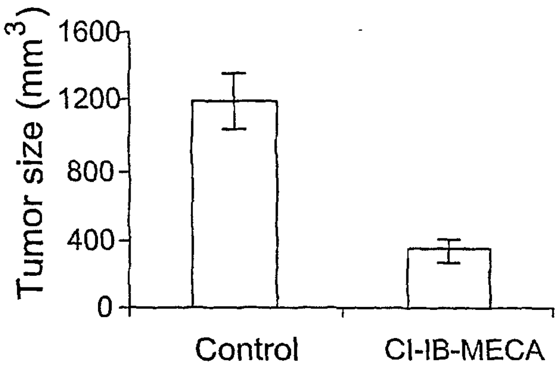


FIG. 5B

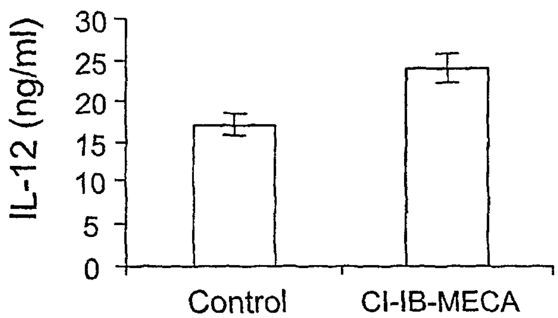


FIG. 5C

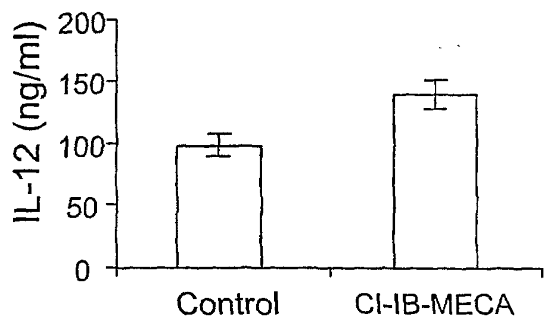


FIG. 5D

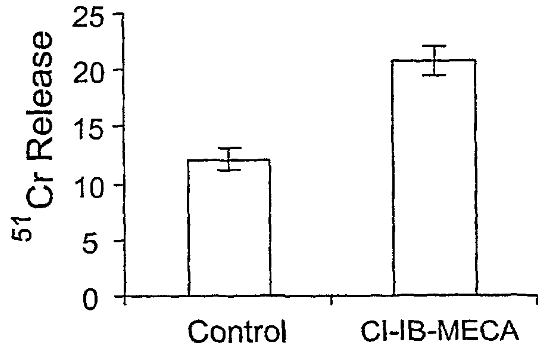


FIG. 5E

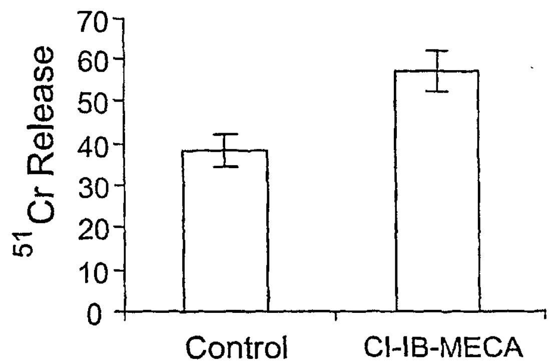


FIG. 5F

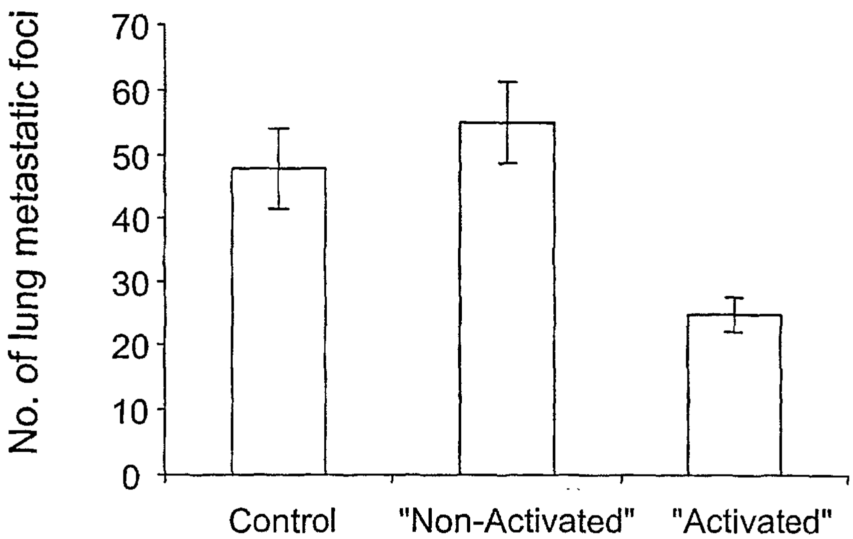
No. of lung metastatic foci
70
60
50
40
30
20
10
0
Control
"Non-Activated"
"Activated"

FIG. 6